Europäisches Patentamt

European Patent Office

Office européen des brevets

(11) Publication number: **0 107 408**
A2

(12) **EUROPEAN PATENT APPLICATION**

(21) Application number: 83305949.6

(22) Date of filing: 30.09.83

(51) Int. Cl.³: **C 12 P 7/06, C 12 F 1/00**

(30) Priority: 30.09.82 US 430782
12.09.83 US 530621

(43) Date of publication of application: 02.05.84
**Bulletin 84/18**

(84) Designated Contracting States: **FR GB IT**

(71) Applicant: **Albers, Walter Frank, 2626 E. Arizona Biltmore Circle, 23 Phoenix Arizona 85016 (US)**

(72) Inventor: **Albers, Walter Frank, 2626 E. Arizona Biltmore Circle, 23 Phoenix Arizona 85016 (US)**

(74) Representative: **Collier, Jeremy Austin Grey et al, J.A.Kemp & Co. 14, South Square Gray's Inn, London WC1R 5EU (GB)**

(54) **Process for producing ethanol.**

(57) Ehtanol is produced from biomass in a single plate still by pasteurizing the biomass in the still and distilling water therefrom, fermenting the biomass, and distilling off the ethanol produced. The residual biomass my be concentrated, in the same still, to produce a dried feed. The aqueous ethanol obtained may be concentrated by treatment with a dehydrating agent and distillation. The dehydrating agent is preferably regenerated and re-used.

0107408

- 1 -

PROCESS FOR PRODUCING ETHANOL

This invention relates to a process which can employ solar or waste heat for the production of ethanol and dried feed. In one aspect, the process of the present invention provides a low scale technology for pasteurization and fermentation of a biomass combined with aqueous ethanol removal, preservation of the spent mash into a dried feed, and simple dehydration and distillation of the aqueous ethanol to fuel grade ethanol while recovering the dehydrating agent. The present invention may employ simple solar stills which, excluding an inexpensive plastic cover, may be economically constructed from native materials. The entire process may be conducted in only two stills without complex plumbing and instrumentation. Furthermore, a dehydrating agent for use in this invention can be made locally from wood ashes. The process is leisurely and may be left unattended. Process stage changes are generally automatic and visibly discernable, thus operators need only minimal skills.

The present practices of pasteurizing and fermenting a biomass to provide an ethanol-water mixture and a spent mash, dewatering the spent mash to provide a dried feed, and concentrating the ethanol-water mixture to provide fuel grade or absolute ethanol are well known in the art. These practices are, however, generally commercially practiced in expensive and sophisticated operations which are capital and energy intensive. For example, pasteurization and fermentation are often carried out in a vessel surrounded by a heating jacket that is heated by high temperature steam which has been generated at high energy cost. Also, simultaneous ethanol removal so as to enhance the efficiency of fermentation may be effected by using expensive vacuum or membrane technologies. Even drying systems for dewatering spent mash generally rely on relatively expensive mechanical compression devices which force water from the mash which then

- 2 -

0107408

may be further dried by heating in a jacketed vessel or by using a forced hot air system, both of which are energy intensive.

It is well known that the aqueous ethanol mixture obtained from the fermentation process requires further processing to remove water therefrom to increase the ethanol concentration. Concentration of ethanol-water mixtures to fuel grade ethanol by distillation is well known. In commercial practice, of the total energy used in ethanol production, normally about one-half is used for distillation. Generally, a distillation column with 20 to 80 evaporations and condensations (each a so-called theoretical plate) achieves the difficult separation of water and ethanol.

In short, there is a need for a lower scale technology to produce concentrated ethanol and dried feed products that offer simplicity of operation and cost savings. This need is especially great for smaller scale ethanol production plants useful in rural areas and in developing countries. It is important to reduce the cost of the energy required for these processes, for example, by utilizing solar energy.

Solar stills are known although usually for the extraction of fresh water from sea water rather than for ethanol concentration. U.S. Patent No. 4,286,066, August 25, 1981, to Butler et al, and U.S. Patent No. 4,314,890, February 9, 1982, to Beck et al, have described the use of solar energy as an alternate or secondary heat source for conventional distillation columns. An inclined apparatus with internal condensation pipes and a glass cover to admit solar energy for distillation of ethanol and other liquids is disclosed in U.S. Patent 102,633, May 3, 1870, to Wheeler et al. However, a simple solar still provides only a single evaporation and condensation (one theoretical plate). Thus, a number of solar stills in series are required to achieve concentrated ethanol. Perhaps as a result of this limitation of simple solar stills, in a recent report (U.S. Department of Energy contract DE-ACO 676 RLD 1830) the Battelle Memorial Institute excluded solar distillation from its discussion of potential energy improvements in ethanol-water separation processes.

Distillation techniques are not the only means by which ethanol concentration can be achieved, however.

Dehydration techniques for ethanol such as the preparation of anhydrous ethanol utilizing anhydrous potassium carbonate have been described as long ago as 1796 (A. Lowita, Crell's Chemical Annals, 1-22 (1796). Also, it is known that dilute solutions of ethanol in water can be split into an aqueous phase and ethanol phase by adding substantial amounts of certain salts which results in a separation into a heavy bottom liquid layer containing mostly salt in water and a top liquid layer containing ethanol and often some water and salt. Ethanol-water separation using complex equipment and high temperature processes to dehydrate a concentrated ethanol-water mixture is disclosed in U.S. Patent No. 1,744,503, January 21, 1930, to Ricard, and U.S. Patents No. 1,879,847, September 27, 1932, and No. 1,891,593, December 20, 1932, both to A. Gorhan. Further development of these techniques was not suggested in the Battelle report which, instead, suggested a method using heated, dried and crushed grain subjected to high temperature ethanol water vapors, taking advantage of the property of starch to preferentially absorb water in the presence of ethanol.

A straight forward economical process combining the processes of pasteurization, fermentation, ethanol removal during and after fermentation, and spent biomass preservation within a single vessel has heretofore been unknown. A practical and economical process for fuel grade or higher grade ethanol production using a chemical dehydrating agent in a low cost still with only one theoretical plate and the ability to use direct solar, low grade or high temperature heat while recovering the dehydrating agent has also heretofore been unknown. It is believed that the present invention provides such processes and hence, uniquely responds to an economic need highlighted by the increasing costs of energy and increasing requirements for practical fuels.

SUMMARY OF THE INVENTION

In accordance with the present invention, ethanol is provided by pasteurizing a biomass, fermenting the biomass, and separating an ethanol-water mixture from the biomass which may be further dried to an animal feed, all in a single vessel. The ethanol-water mixture may then be subjected to distillation in a second still which contains a dehydrating

- 4 -
0107408

agent or counter current extraction using the dehydrating agent which is regenerated by removing a portion of water therefrom and reused. The stills are suitably heated by solar heat or low grade waste heat, although heat from other sources may alternatively be used.

## BRIEF DESCRIPTION OF THE DRAWINGS

Figure 1 is a schematic of the process steps of the present invention illustrating a biomass which is fermented, distilled and then dried, and illustrating the concentration of ethanol and recovery of the dehydration agent;

Figure 2 is a somewhat schematic vertical cross-section of a solar still;

Figure 3 is a somewhat schematic vertical cross-section of a still that accepts bottom heat;

Figure 4 is a somewhat schematic cross-section of a multi-effect still.

## DETAILED DESCRIPTION OF THE INVENTION

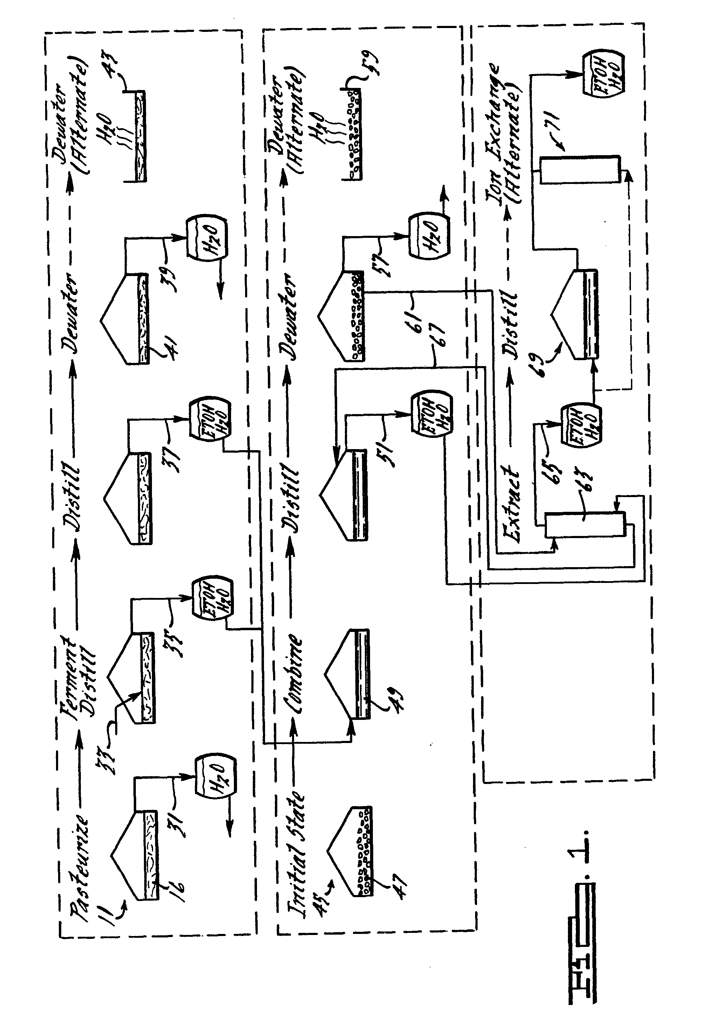

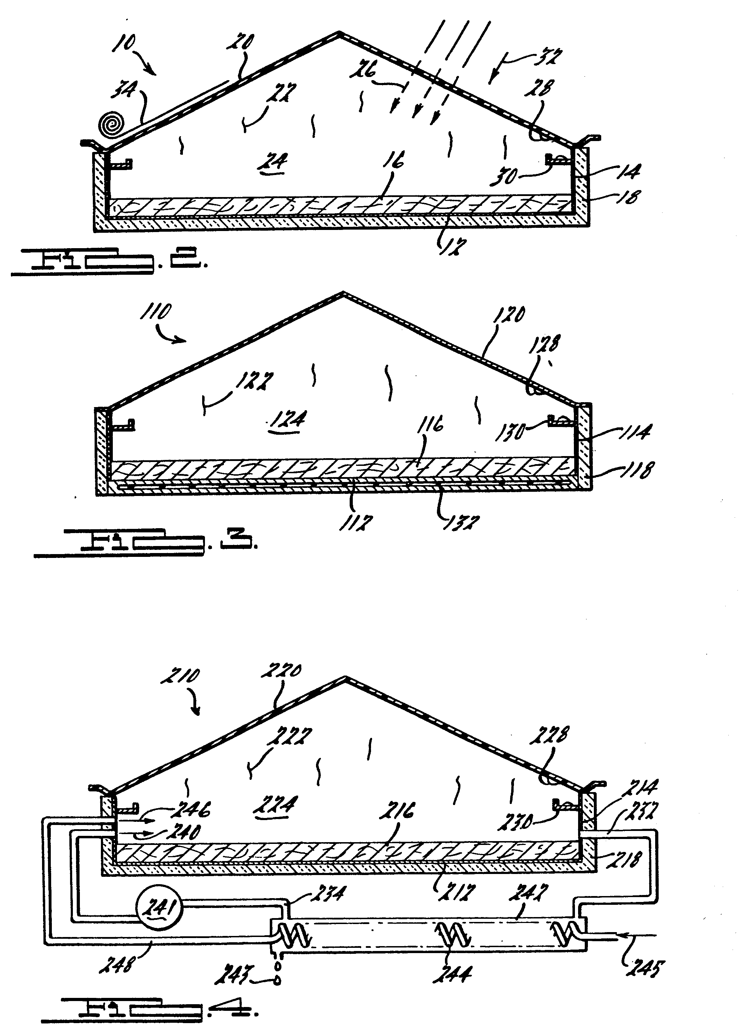

Now referring to the figures, Figure 1 illustrates the process steps of the present invention while Figures 2 through 4 illustrate three types of stills which can be used to carry out the process steps of the invention. Broadly speaking, the process of the present invention involves, in a first still, distilling aqueous ethanol from a fermenting or fermented biomass or other ethanol containing material and subsequently dewatering the biomass to provide a dried feed and, in a second still, increasing the ethanol concentration of aqueous ethanol by distillation with a dehydrating agent and subsequently dewatering the agent to regenerate it for reuse in the process. The process of the present invention is particularly suitable for stills having a single plate by which is meant stills adopted for simple distillation. In such stills, there is no appreciable rectification of the vapor formed from the liquid in the still. It will, of course, be appreciated that the aqueous ethanol mixture may contain minor amounts of other components. It will also be appreciated from the following disclosure that the process of the present invention is subject to variations and modifications which will be within the skill of those in the art and which are contemplated to be within the broad scope of the present invention.

Figure 2 is a somewhat schematic vertical cross sectional view of a solar still, indicated generally by the numeral 10, having a base 12 with upwardly extending walls 14 defining a container which holds biomass 16 to be heated or heated and distilled. It will be appreciated that, broadly speaking, biomass 16 can be any ethanol-containing material. Walls 14 are surrounded by insulation 18 which should provide thermal insulation equivalent to about one inch of polystyrene foam. Solar still 10 has a light admitting sloping cover 20 which can be transparent plastic film, or alternatively glass, and can have a greenhouse shading curtain 34. Cover 20 has four functions. First, cover 20 isolates vapors 22 within interior 24 of still 10 from outside air. Second, cover 20 admits sunlight radiation 26 into interior 24 to heat biomass 16 and base 12. Third, cover 20 is in contact with and is cooled by outside air and thus provides a cool surface upon which vapors 22 condense. And fourth, cover 20 slopes from the horizontal, at least 7 degrees, to provide means for collecting condensed liquid 28 which flows on the undersurface of cover 20 down to trough 30 and then exits from the still. It will be appreciated that still 10 operates in a conventional manner with radiant energy from the sun passing through cover 20 in direction 32 to heat the interior of still 10 but not cover 20 itself, which provides a relatively cool surface relative to the other surfaces of the still for condensation of distilled vapors thereon. Cover 20 can be made of plastic or glass, but plastic covers are preferred for reasons of economy. Although glass is water wettable, a plastic film modified to be water wettable on its lower side is most preferred as offering the best performance.

Figure 3 is a somewhat schematic vertical cross-sectional view of a type of still, indicated generally by the numeral 110, that utilizes bottom heat. Bottom heat is supplied to the base of the still and can be low temperature heat found, for example, in liquids or gasses that have been used in cooling industrial processes or electrical generating plants or it may be supplied from solar collectors. In addition, this heat may be partially supplied by brine heated in various processes of this invention. Also, this bottom

0107408

heat may be supplied by high grade heat that is generally thought to be high temperature heat such as can be provided by a combustion process or the like or a concentrating type solar collector. Still 110 has a base 112 with walls 114 extending upwardly therefrom to define a container for biomass 116 to be heated, or heated and distilled. Walls 114 have insulation layer 118 which should provide thermal insulation equivalent to or greater than one inch of polystyrene foam. Still 110 has a sloping cover 120 that can be made of any heat conductive material with a water wettable undersurface. Cover 120 entraps vapors 122 within interior 124 of still 110, thus trapping heat within the still but cover 120 is itself cooled by outside air and, hence, provides a cool undersurface upon which vapors 122 condense to provide distillate 128 which flows down to trough 130 and exits from the still. Base 112 and biomass 116 are heated by heat from an external heat source which is transferred thereto through a heat exchanger 132 located within or below base 112 or within biomass 116. Alternatively, it is contemplated that the heat source for still 110 can be both bottom heat and solar energy. Of course, this alternative requires that cover 120 be transparent to admit sunlight into still 110.

It is well known that the above stills are single-effect thermal devices primarily because of the conductiveness of covers 20 and 120. In order to improve the energetic efficiency, each stage of the distillation cycle may be optimized as well as some of the energy recycled. Therefore, the functions of the stills may be segregated such that a separate unit utilizing solar or bottom heat is used to raise the temperature of the substance to be distilled, the liquid vaporized in an evaporation unit, and the distillate collected in a separate condenser device. The heat released during condensation may be used to heat additional evaporation units and/or may be used to heat the substances to be distilled. This reuse of heat is commonly employed and is generally termed multi-effect distillation.

Figure 4 is a somewhat schematic cross-sectional view of one type of still, indicated generally by the numeral 210 that allows separation and performance optimization of the still functions. In addition, some of the collected energy is reused which allows this type of still to be termed a

0107408

multi-effect device. Still 210 has a base 212 with walls 214 extending upwardly therefrom to.define a container for biomass 216 to be heated, or heated and distilled. When solar energy is used the base and walls have insulation layer 218 which should provide thermal insulation equivalent to or greater than one inch polystyrene foam. Solar still 210 has a light transmitting sloping cover 220 which can be one or more layers of a transparent plastic film, or alternatively glass. Cover 220 entraps vapors 222 within interior 224 of still 210, thus trapping heat within the still. The vapors 222 are moved by air 240 from fan 241 across the still where the vapor 222 and air mixture from the still interior exits the still through conduit 232 and is passed into condenser 242. In condensor 242 vapors condense providing distillate 243 while air 240 and vapors which did not condense are returned through conduit 234 to the still. Air 240 causes circulation inside still 210 to avoid stratification of air and vapors 222 therein. The condenser coils 244 are cooled by still feedstock 245 which increases in temperature by absorbing the released heat of condensation from the vapors 240 and then passes through conduit 248 and enters the still as indicated by arrow 246. The still may additionally be equipped with trough 230 which collects distillate 228 from the underside of cover 220.

The foregoing stills may be looked upon as offering a range of skill required for operation, unattended or attended operation, and capital investment. Solar stills offer the "least cost" alternative for each of these criteria but deliver variable output dependent upon solar energy received, and lack functionality in certain geographic areas. A still utilizing waste heat offers a steady output and is in the lower range of the above criteria but requires, and must be located near, a source of low grade heat. A high temperature heat multi-effect still provides the highest output per land area utilized, is more location independent, and offers the possibility of incorporation into existing conventional distillation plants; but is at the high end of the complexity, operational and investment range.

A preferred process of the present invention is illustrated schematically in Figure 1. Thus, a pulverized biomass 16 suitable for fermentation is heated in a still indicated by the numeral 11 which can be of the construction of still 10 or alternatively, still 110 or 210, to between about 65°C up to its boiling point to pasteurize the biomass and vaporize some of the excess water therein. The water is distilled and can optionally be collected as indicated by numeral 31 for recycling in the process or for other use.

In the same still, yeast acting materials are added at numeral 33 to the pasteurized biomass which is heated for fermenting of the biomass. The temperature of the biomass is maintained sufficiently high to maintain fermentation which is normally not above 44°C. The temperature can be controlled by means of a greenhouse shading curtain 34 for solar still 10 or by adjusting the flow of bottom heat for still 110. Concurrent with fermentation, gaseous ethanol and water separate from the fermenting biomass, condense onto the cool surface of covers 20, 120, or condenser coils 244 and are collected as indicated at numeral 35.

After fermentation is completed, if ethanol remains in the fermented biomass, continued distillation first yields a condensate of ethanol and water as indicated by numeral 37 and then of substantially pure water as indicated by numeral 39 thus dewatering the perishable biomass or spent fermentation mash and transforming it into a dried preserved animal or human grade food 41. The temperature of the biomass may be raised to at least 50°C up to the degradation point of the biomass during the dewatering step. Following the removal of the ethanol-water mixture from the biomass, in warm arid climates dewatering may alternatively take place in uncovered, open vats, such as vat 43.

A second still, indicated by the numeral 45 which can be of the same construction as still 10, 110 or 210, can then be employed to increase the ethanol concentration of aqueous ethanol which can be that of numerals 35 and 37 obtained from the first still. In the initial state, the second still contains a dehydrating agent 47 of the present invention. Aqueous ethanol collected from the fermentation and distillation of the biomass in the first still or alternatively any other aqueous ethanol mixture is added to the dehydrating agent containing still to form an aqueous composition 49. The dehydrating agent is hydrophilic in nature and preferably attracts or binds a substantial portion of the water in the ethanol-water mixture, thus increasing the ethanol concentration in the remaining ethanol-water mixture. The concentrated ethanol water mixture is then subjected to distillation for further separation to provide a further concentrated ethanol in water mixture as indicated by the numeral 51; or alternatively, if the concentrated ethanol-water mixture separates out as a separate upper phase from the water-combining agent mixture, then it may be either distilled or directly withdrawn. However, since a portion of the ethanol-water mixture generally remains in the lower phase which then must be distilled, directly withdrawing the upper phase has minimal, if any, process advantage.

Where the ethanol-water mixture of increased concentration is removed from the brine by distillation, the temperature of the concentrated ethanol-water mixture may be raised to at least 30°C up to its boiling point in the still to obtain a gas further enriched in ethanol content which contacts relatively cool surface of covers 20, or 120, or condenser 242 for collection of a distillate.

After the concentrated ethanol-water mixture has been removed from the brine, the dehydrating agent is regenerated by removing a portion of the water from the brine as indicated by the numeral 57. The temperature of the brine can be increased to remove water from the brine. Water so removed may be collected as distillate and optionally may be reused with a biomass for fermentation or any other desired use. After removal of some of the water from the brine, the dehydrating agent is regenerated and can be recycled to the initial state. In warm arid climates the dewatering process may take place in uncovered open vats 59 or may take place in open ponds similar to those used for sea salt production followed by return of the dried dehydrating agent to the still. It should be noted that these open ponds also may be constructed as and function as gradient or saturated solar ponds and supply heated brine mixtures to stills utilizing low grade heat or to evaporation devices. It should be appreciated that not all of the water need be removed from the brine in order to recycle the dehydrating agent, but sufficient water must be removed so that the agent can effectively function to dehydrate the aqueous ethanol.

Further concentration of the ethanol may be necessary particularly if the ethanol is to be mixed with

0107408

gasoline or used in certain chemical processes. If the dehydration agent in use does not provide a phase separation then the ethanol-water mixture 51 may be returned to still 45 containing the reconstituted agent 47 and the distillation, and dewatering steps repeated. However, by using a dehydrating agent that provides for phase separation, further ethanol concentration can take place outside the still in a simple counter flow extraction tower 63 where the dehydrating agent 61 from the dewatering still or the solar pond enters near the top of the tower and the aqueous ethanol 51 enters near the base of the tower. The aqueous ethanol and the dehydrating agent flow counter to each other with a concentrated ethanol water mixture 65 exiting at the top of the tower and the diluted dehydrating agent 67 flowing from near the tower base. This diluted brine contains some ethanol and is subjected to distillation in the solar still. Also, the concentrated ethanol-water mixture 65 generally contains some concentration of the dehydrating agent. The dehydrating agent is preferably removed, especially if the ethanol is to be used as an internal combustion engine fuel or as a solvent. Therefore, preferably the concentrated ethanol-water mixture 65 is distilled in still 69 or the dehydrating agent otherwise removed utilizing ion exchange techniques in ion exchange column 71. Suitable ion exchange apparatus and resins will be apparent to those skilled in the art. For example, if potassium carbonate is used as the dehydrating agent, Rohm and Haas IR120H beads may be used.

Suitable dehydrating agents for use in the present invention preferentially attract or bind water relative to ethanol. The dehydrating agents suitable for use herein are: (1) chemically stable, (2) separate easily from water, (3) are not corrosive under the conditions of use, (4) do not react with ethanol, (5) are not vulnerable to bacterial attack, (6) are highly soluble in water, and (7) are readily available at low cost. Such agents include salts which have a solubility in water and which do not react with ethanol under the conditions of use, such as water soluble salts of weak acids. Suitable agents include water soluble salts, especially the alkali metal, calcium, barium and magnesium salts of boric acid, carbonic acid, acetic acid, propionic acid, thiosulfuric acid, citric acid, tartaric acid, and mixtures thereof. One

- 11 -

0107408

preferred dehydrating agent is a mineral salt with a solubility in water greater than 30 grams per 100 grams water. Other preferred agents include the salts potassium carbonate, potassium sodium carbonate, sodium carbonate, potassium acetate and sodium acetate. These salts, including mixtures thereof, function well in solar stills and low grade heat stills as water binders. As potassium carbonate ("pot ash") can economically be made from wood ashes, it is the most preferred salt for use in developing countries.

In accordance with the present invention, production of fuel grade ethanol has been accomplished in solar stills in which two distillations in series, the output of one still becoming the input of the next still, has raised the ethanol concentration of a feedstock from 11.8 percent to 93.4 percent by volume. In a test utilizing high grade heat, a 4 percent feedstock was increased to 91 percent using a still with one theoretical plate. It should be especially noted that, in contrast to conventional distillation processes using distillation columns, in the process of the present invention, the amount of energy required to increase the concentration of ethanol in an aqueous ethanol mixture decreases as the ethanol concentration increases.

The following Examples were carried out utilizing the processes described above and are illustrative of the invention but are not to be considered as limitations thereof. It is apparent from the following Examples that each step of the process may take place in its own still and the process made continuous.

0107408

## Example 1

Solar pasteurization of fermentable raw material was carried out as follows. Artificially ripened commercially available bananas were used. The solar still was constructed of galvanized steel and was 25 cm square with a basin area of .0625 square meter. The front wall measured 2 cm in height and the rear 10 cm. The collection trough was adjacent to the front wall. The distillate outlet was located below the trough and the solution inlet was located in the rear wall. Exterior walls and base were surrounded with 2.5 cm thick polyurethane insulation. The still was covered with light transmissible 0.1 mm plastic (polystyrene) film with the underside treated to provide a wettable surface. 750 ml of whole bananas were ground in a food pulverizer (with outlet holes measuring 0.5 cm), loaded into the vat, and placed in direct sunlight for one day.

The temperature within the solar vat was adequate for pasteurization at 72 degrees C in several hours. Liquid collected via the solar still collection trough was returned to the still in order to maintain volume at 750 ml.

## Example 2

The product of Example 1 was fermented and simultaneously distilled as follows using a shallow still and solar energy. The residual solution (750 ml ground bananas with peel) from Example 1 remained in the same solar still over which a shading curtain commonly used in greenhouse operation was placed to control the solar energy. A prepared yeast (dry bread and sherry wine yeasts) suspension (30 ml) was added to the 750 ml banana mixture in the evening when the mixture had cooled to 38 degrees C. Distillation began the next morning.

Distillate collections were as follows:

| Period | Distillate Collected | Volume Percent Ethanol | Ml Ethanol | Highest Temperature Within Vat |
|--------|---------------------|------------------------|------------|-------------------------------|
| 1st day | 97 ml | 7% | 6.79 | 38 degrees C |
| 2nd day | 115 ml | 9% | 10.35 | 40 degrees C |
| 3rd day | 154 ml | 9% | 13.86 | 44 degrees C |
| 4th day | 120 ml | 8% | 9.60 | 44 degrees C |
| 5th day | 70 ml | 6% | 4.20 | 44 degrees C |
| 6th day | 20 ml | 2% | 0.40 | 46 degrees C |
| Total | 576 ml | 7.8% | 45.20 | |

Further distillate collections revealed no significant ethanol content.

The 4 mil plastic film covering the still allowed transfer of the heat generated by the exothermic ethanol fermentation. Initial fresh air for yeast growth was provided by the vat inlet, which later provided for $CO_2$ escape. Distillate began collecting within several hours of sun exposure and $CO_2$ bubbles appeared on the surface of the liquid for the first four days. By comparison, a 750 ml slurry of whole bananas was pasteurized by energy expensive conventional means and allowed to ferment in a vented container with mixing provided three times daily. The distillate of this solution yielded an ethanol concentration of 7.6 percent.

## Example 3

The product of Example 2 was treated further to yield a dried feed by drying in the same still as in Example 1. The residual solution of whole bananas from Example 2 remained in the same shallow still. The shading curtain was removed. Drying took place by solar evaporation.

In two days water output totaled 25 ml. The dried bananas weighed 291 grams with a moisture content of 15 percent.

## Example 4

Ethanol was distilled from a fermented corn mash by use of a solar still in accordance with the following. Industrially fermented corn mash with an ethanol content of approximately 8 percent was used. The solar still was constructed of a 2.54 cm thick wooden base placed over 2.54 cm

thick polyurethane insulation. The wood sides were 10 cm high with two opposing sides containing 1.26 cm aluminum channel condensation collection troughs mounted at a 4 percent slope. The basin liquid reservoir (0.836 square meters) was 0.159 cm aluminum sheeting covered with a heat absorbent black pigmented paint. Solution inlets and drains were constructed over the basis sideboards with a side inclination of 50 degrees. The light transmissible cover was 0.1 mm PVF film (polyvinyl fluoride). Eight liters (7,504 grams) of corn mash was loaded into the still early one morning. Distillate appeared in the collection troughs that afternoon.

Distillate collections are presented below:

| Period | Distillate Collected | % Ethanol By Volume | Amount Ethanol | Basin Temperature °C |
|---|---|---|---|---|
| 1st day | 565 ml | 13% | 74 ml | N/A |
| 2nd day | | | | rain |
| 3rd day | 1,960 ml | 13% | 255 ml | 60 degrees |
| 4th day | 1,590 ml | 12% | 191 ml | 58 degrees |
| 5th (noon) | 515 ml | 10% | 52 ml | 58 degrees |
| 5th (7:00 pm) | 950 ml | 5% | 48 ml | 44 degrees |
| 5th (9:00 pm) | 60 ml | 0-1% | 0 ml | 36 degrees |
| Total | 5,731 ml | 11% | 620 ml | |

620 ml ethanol collected by distillation divided by 8,000 ml mash liquid yielded an ethanol percent of 7.75 by volume which compares favorably to the original 8 percent in the mash feedstock.

## Example 5

Solar dried feed was obtained from the product of Example 4 as follows. The residual solution of distillers grain and water from Example 4 remained in the same shallow still and was dried by solar evaporation. Water output totaled 545 ml the first day and 20 ml the next day. The distiller's dry grain removed from the vat weighted 1,077 grams. An average sized piece measured 4 x 5 x 0.8 cm. An independent test yielded a moisture content of 14.1 percent. This material was stored at 17 to 21 degrees C for over four months with no apparent deterioration.

## Example 6

Fuel grade ethanol was produced from dilute ethanol using a solar still containing potash. The potash used was industrial grade 99 percent $K_2CO_3$. The solar still was constructed of galvanized steel and measured one meter square. The slope of the solar roof was 12 degrees with the rear wall 21 cm high and the front 3 cm high. A collection trough 2 cm wide was positioned parallel to the front wall and was provided with four connected outlets. Still loading was accomplished by means of a rear inlet connected to a distribution pipe. The basin was coated with a heat absorbent black paint while all exterior metal surfaces were covered with 2.54 cm polyurethane foam insulation. The light transmissible cover was a 0.318 cm standard double strength window glass.

In the evening, 8 liters of potash (weighing 9.8 kilograms) was mixed with 4 liters of tap water (16 degrees C) and loaded into the solar still. Eight liters of tap water containing 950 ml of ethanol (an 11.8 percent concentration) were added over the surface of the potash solution. This method utilized the properties of potash to increase the surface concentration of ethanol in the basin which in turn would be vaporized and collected. Solar energy was used as the heat source and distillation began the following morning.

Outputs collected were as follows:

| Time | Distillate Collected | Volume Percent Ethanol | Ml Ethanol | Basin Temperature |
|------|------|------|------|------|
| 11:00 am | 600 ml | 82% | 492 ml | 52 degrees C |
| 12:15 pm _"A"_ | 550 | 71 | 291 | 59 degrees C |
| 12:25 | 65 (1) | 42 | 27 | |
| _"B"_ | 65 | 26 | 17 | |
| | 65 | 15 | 10 | |
| 1:00 | 65 | 3 | 2 | 72 degrees C |
| | 65 | 3 | 2 | |
| | 65 | 2-3 | 2 | |
| 2:00 | 65 | 1 | 1 | |
| Total | 1,605 ml | 58.8% | 944 ml | |

(1) Smallest sample measurable by hydrometer.

0107408

A total of 99.4 percent of the ethanol was recovered with a rise in concentration from 11.8 to 58.8 percent. At point "A" 95.8 percent of the ethanol had been removed at a 75 percent concentration and at point "B", 98.6 percent at a 70 percent concentration.

## Example 7

The potash solution used in Example 6 was recycled as follows. The residual from Example 6 (a mixture of 10.4 liters of water and 9.8 kilograms of potash) was left in the same shallow still. Output of the still was allowed to continue to reduce the potash solution to the original concentration which contained 4 liters water. The water removed is shown below:

| Day | Distillate Collected | Basin Temperature °C | Observations |
|---|---|---|---|
| Same as #5 | 1,300 ml | 70 degrees | |
| 1st | 940 ml | 70 degrees | 18" knife like crystal formations |
| 2nd | 35 ml | (rain) | |
| 3rd | 1,255 ml | 91 degrees | |
| 4th | 925 ml | | crystal mounds forming |
| 5th | 930 ml | 74 degrees | |
| 6th | 875 ml | | 2/3 of vat with crystals |
| Total | 6,260 ml | | |
| Water remaining | 4,160 ml | | |

## Example 8

Potash was recrystallized from a brine solution using a solar still as follows. The residual solution from Example 7 (a mixture of 4.16 liters water and 9.8 kilograms potash) was left in the same shallow still and solar dried. Over a four day period, an additional 1.9 liters of water was removed. The resultant solar dried potash (large crystals and chunks) was found to contain 19% water corresponding to $K_2CO_3$ · 1.8 $H_2O$.

## Example 9

Evaporation rates of a solar still and a solar evaporation pond were compared. The still was constructed of stainless steel surrounded by 2.5 cm foam insulation. The still was lined with a 7 mil black plastic film and measured 50 by 25 cm with a surface area of .125 square meter. The still cover was double strength window glass. The pond was built of 2.5 cm foam insulation with the same black plastic film liner and measured 30 cm by 24 cm giving a surface area of .072 square meter. The still was loaded with 3 liters of a 2 to 1 volume ratio of water and potassium carbonate and the pond contained 7 liters of the same mixture. The still and pond were replenished each day.

Outputs were collected and averaged over partly cloudy days with high wind activity and clear days with low wind activity. The results were presented below:

|  | Still | Pond |
|---|---|---|
| Cloudy Weather |  |  |
| Maximum Basin Temperature (C) | 81 | 47 |
| Output (liters per square meter) | 1.68 | 4.40 |
| Percent Still Output to Pond Output | 38 |  |
| Clear Weather |  |  |
| Maximum Basin Temperature (C) | 97 | 52 |
| Output (liters per square meter) | 3.02 | 6.25 |
| Percent Still to Pond | 48 |  |

## Example 10

The output from a previous distillation was redistilled to increase the ethanol concentration as follows. The 12 kilograms of solar dried potash crystals of Example 8 were left in the same solar still. The 783 ml output of Example 6 (75 percent ethanol-point "A") were loaded in the vat in the evening. Solar energy was used as the heat source and distillation began the following morning. Outputs collected were as follows:

| Time | Distillate Collected | Volume Percent Ethanol | Ml Ethanol | Basin Temperature |
|------|------|------|------|------|
| 11:00 a.m. | 282 ml | 93% | 262 ml | 62° C |
| 12:00 _"C"_ | 292 _ _ _ | 94 | 274 | 67° |
| 1:00 p.m. | 57 | 75 | 43 | 63° : |
| 2:00 | 16 | 30 | 5 | 60° |
| 3:00 | (8) | 0 | 0 | N/A |
| Total | 647 ml | 90.3% | 584 ml | |

A total of 99.5 wt% of the ethanol was recovered with a rise in concentration from 75 to 90.3 percent. At point "C" 91.8 wt% of the ethanol had been removed with a concentration of 93.4 wt%.

## Example 11

A comparison of concentrations of ethanol distillate were made using different concentrations of potash and water. The solar still was made of stainless steel and measured 500 by 250 cm. The slope of the roof was 15 degrees. A 3 cm wide collection through was positioned parallel to the front wall and provided with two connected outlets. Black polyethlene film of 0.2 mm thickness lined the basin and polyurethane foam of 2.54 cm thickness insulated all exterior metal surfaces. The light transmissible cover was single-strength window glass.

Each morning a premixed solution consisting of 1.5 liters potash weighing 1.84 kg plus 1.5 liters water was loaded into the still. Then a 10 percent ethanol-water mixture was added so that the total volume ratios of liquid to potash were as listed below. The distillate sample collected was equal to the amount of ethanol added to the still.

Serial distillate samples were then collected as listed below:

0107408

| Serial Number | Volume Liquid to Volume Potash Ratio | Approximate Potash Weight Percent | % Ethanol By Volume |
|---|---|---|---|
| 1 | 1.50 : 1 | 82% | 78% |
| 2 | 1.67 : 1 | 73% | 69% |
| 3 | 2.00 : 1 | 61% | 58% |
| 4 | 2.17 : 1 | 56% | 50% |
| 5 | 2.32 : 1 | 53% | 38% |
| 6 | 2.91 : 1 | 42% | 31% |
| 7 | 4.89 : 1 | 25% | 27% |
| 8 | 12.22 : 1 | 10% | 23% |
| 9 | No Potash | 0 | 19% |

### Example 12

An ethanol solution was concentrated by phase separation using solar dried crystalline potash of Example 8 which was subsequently reconstituted in the same still in accordance with the following. The 12 kilograms of solar dried potash crystals of Example 8 were left in the same solar still. Twelve (12) liters of a 10 percent ethanol-water solution were added and the mixture allowed to stand in the sun for one day with the collection trough cock closed. The mixture temperature increased from 20 degrees to 58 degrees C thereby increasing the solubility of the potassium carbonate mixture. That evening the concentrated ethanol solution that was layered above the potash-water solution was drained through the side of the still. Some overflow of the water-potash mixture was also collected and was separated from the ethanol water mixture by means of a separation funnel and returned to the still. The potash solution (including some 2.3 kilograms of potash crystals not in solution) was reconstituted as in Example 7.

The ethanol-water solution measured 1.464 liters and contained 81 percent ethanol by volume and 19 percent water representing a 98.8 percent recovery of the initial ethanol. In a determination 46.3 grams of the 81 percent ethanol-water solution was evaluated and found to have a residue potassium carbonate content of about 300 ppm. No potassium carbonate was found in a 200 ml sample of the 81 percent ethanol-water solution which had been solar distilled.

## Example 13

Various amounts of water, ethanol, and potassium carbonate were combined to determine which combinations are compatible and which result in separation into two layers permitting collection of a top aqueous ethanol layer and a lower aqueous potassium carbonate layer. The temperature of the chemicals approximated ambient temperature or $25^{\circ}C$.

Serial combinations containing the approximate minimum concentration of potassium carbonate to achieve separation are listed below:

| Serial Number | Ethanol Per 100 Parts Water | Potassium Carbonate Per 100 Parts Water |
|---|---|---|
| 1 | 5 | 44 |
| 2 | 10 | 38 |
| 3 | 20 | 31 |
| 4 | 38 | 17 |
| 5 | 49 | 14 |
| 6 | 61 | 1 |

## Example 14

A portion of the distillate from Example 6 (58.8 volumes percent or 53 percent by weight) was subjected to counter flow extraction to further concentrate the ethanol. The extractor consisted of four (4) 250 ml beakers (equal to an extractor of four stages) resting on stirring devices. Each beaker contained 22 grams potash and 20 grams water. 21.2 grams ethanol mixed with 18.8 grams water was added to the first beaker. The resultant top layer was removed and passed to the second beaker, with the sequence repeated until the top phase from the third beaker was placed in the fourth beaker. In order to achieve approximate steady state conditions, the above sequence was cycled four times by discarding the lower phase contents of the first beaker and adding a fourth beaker each cycle.

The approximate steady state of materials leaving each stage was as follows:

- 21 -                                              0107408

| Stage | Ethanol Phase | | | Salt Phase | | |
|---|---|---|---|---|---|---|
| | Wt% | Ethanol | Water | Potash | Ethanol | Water |
| 1 | 71 | 20.2g | 7.9g | 22.2g | 2.7g | 37.1g |
| 2 | 86 | 19.9 | 3.1 | 22.2 | .74 | 26.2 |
| 3 | 90 | 19.7 | 2.3 | 22.2 | .44 | 21.4 |
| 4 | 92 | 19.5 | 1.7 | 22.2 | .20 | 20.6 |

### Example 15

A 3.1 wt% ethanol commercial light beer was distilled in a simple one plate still containing potash. 350 ml light beer and 320 ml dry potash were used. A still, having substantially one theoretical plate and providing no reflux, was assembled from a conical-shaped glass distillation pot (Erlenmeyer type) with a side arm. The still was insulated with metal foil to prevent re-evaporation of the distillate. The arm was connected to a simple water cooled condenser-collector tube. The still was heated with either hot water or hot oil as required. The potash was placed in the still. After mixing in the beer the still was heated.

Ethanol distilled off at a pot temperature of 79 degrees C and terminated at 95 degrees C. The output totaled 12 g liquid and contained slightly less than 11 g ethanol or a 91 wt% ethanol concentration.

### Example 16

A single plate distillation of 91 percent ethanol was carried out as follows to concentrate the ethanol in a simple single plate still containing potash and then recover the water from the potash. The materials used were 31.3 g water, 313.0 g ethanol (91 wt%, 94 vol% ethanol) and 125.2 g $K_2CO_3$. The still used was that of Example 12. The ethanol mixture was then added to the still and the temperature remained at 77.8°C for the ethanol distillation and then raised to 105 degrees C for the water distillation. The initial output was 324.1 g ethanol-water mixture containing 312.8 g ethanol (96.5 percent by weight) indicating a 99.9 percent recovery of the initial ethanol. After a break in the distillation, the captured water distilled over. The dried potash was found to weigh 121.8 g.

## Example 17

A single plate distillation of 91 percent ethanol was carried out with potassium acetate to concentrate 91 percent ethanol in a simple single plate still. The materials used were 30.3 g water, 303.4 g ethanol (91 wt% ethanol) and 125.3 g K-acetate. A still containing one theoretical plate (described in Example 12) was used. The K-acetate was placed in the still and the ethanol mixture was added. The pot temperature remained at 77.8 degrees C for the ethanol distillation. No phase separation occurred as K-acetate is very soluble in both ethanol and water. The output fractions are given below:

| Fraction | Weight | Density $(d^{25}_{25})$ | Wt% Ethanol | Weight Ethanol |
|----------|--------|------------------|-------------|----------------|
| 1 | 41.3 | 0.8018 | 95.1% | 39.3 g |
| 2 | 39.9 | 0.8015 | 95.3 | 38.0 |
| 3 | 43.1 | 0.8011 | 95.4 | 41.1 |
| 4 | 40.0 | 0.8008 | 95.5 | 38.2 |
| 5 | 39.4 | 0.8008 | 95.5 | 37.6 |
| 6 | 34.6 | 0.8011 | 95.4 | 33.0 |
| 7 | 35.5 | 0.8020 | 95.1 | 33.8 |
| 8 | 34.5 | 0.8229 | 87.4 | 30.2 |
| Total | 308.3 g | | | 291.2 g |

All Fractions together had an ethanol concentration of 94.5 percent by weight.

- 23 -

0107408

CLAIMS

1. A process for producing aqueous ethanol from a biomass comprising the steps of:

(A) pasteurizing said biomass in a single plate still to provide a pasteurized biomass while simultaneously distilling water therefrom;

(B) fermenting said pasteurized biomass in said single plate still to obtain aqueous ethanol alcohol vapors therein; and

(C) condensing said aqueous ethanol vapors on said single plate to form a first aqueous ethanol condensate and collecting said first aqueous ethanol condensate.

2. A process for producing aqueous ethanol and a dried feed from a fermented biomass comprising the steps of:

(A) heating a fermented biomass in a single plate still to remove residual aqueous ethanol therefrom as an aqueous ethanol vapor;

(B) condensing said aqueous ethanol vapor to form an aqueous ethanol condensate and collecting said aqueous ethanol condensate; and

(C) subsequently further heating said biomass in said single plate still to remove residual water therefrom as water vapor.

3. A process for increasing the ethanol concentration of aqueous ethanol solution substantially free of biomass comprising the steps of:

(A) in a single plate still combining aqueous ethanol with an effective amount of a water soluble dehydrating agent and heating the mixture so formed to provide aqueous ethanol vapors;

(B) in said still, condensing said vapors of step (A) to provide a condensate, said condensate having greater ethanol concentration than said aqueous ethanol of step (A);

(C) in said still collecting said condensate; and

(D) regenerating said dehydrating agent for use in step (A) by heating aqueous composition remaining after step (C) to remove water therefrom.

4. A process for producing concentrated ethanol and dried feed from a biomass comprising the steps of:

(A) pasteurizing the said biomass in a single plate still to provide a pasteurized biomass;

(B) fermenting the said pasteurized biomass in the said single plate still, distilling aqueous ethanol vapours therefrom, condensing the said vapours and collecting the condensate to form a first aqueous ethanol condensate;

(C) further heating the said biomass in the said single plate still to remove residual water therefrom as water vapour and from a dried feedstuff;

(D) combining the said first aqueous ethanol condensate in a single plate still with a dehydrating agent, heating the mixture so formed to produce ethanol vapours and condensing the said ethanol vapours to produce a concentrated ethanol condensate; and optionally

(E) heating the dehydrating agent from step (D) to regenerate it and re-using it in step (D)

5. The process of claim 3 or 4 wherein the said dehydrating agent is regenerated by heating said dehydrating agent in a separate vessel to remove water therefrom.

6. The process of claim 3, 4 or 5 wherein the mixture of first aqueous ethanol condensate and dehydrating agent has an upper layer and a lower layer, the said upper layer having a greater concentration of ethanol than said lower layer, and wherein said upper layer is collected prior to heating the mixture to produce ethanol vapours.

7. The process of any of claims 3 to 6 wherein the ethanol concentration of the said condensate is increased by counterflowing said condensate and a water soluble dehydrating agent.

0107408

8. The process of any of claims 3 to 7 wherein trace amounts of said dehydrating agent are removed from the said condensate by further distilling said condensate, or by passing said condensate through an ion exchange resin.

9. The process of any of claims 3 to 8 wherein the said dehydrating agent is a mineral salt with a solubility in water greater than 30 gram per 100 gram water at the operating temperature of said process.

10. The process of claim 9 wherein the said dehydrating agent is potassium carbonate, potassium-sodium carbonate, sodium carbonate, potassium acetate, sodium acetate or a mixture thereof.

11. The process of claim 1, 2 or 3 wherein additional steps of condensing and collecting water vapour are carried out.

12. The process of any of claims 1 to 11 wherein heating is supplied through the cover of the still or by bottom heat.

13. The process of any of claims 1 to 12 wherein the single plate still is a multi-effect device.

Pasteurize → Ferment Distill → Distill → Dewater — → Dewater (Alternate)

Initial State → Combine → Distill → Dewater → Dewater (Alternate)

Extract → Distill — → Ion Exchange (Alternate)

FIG. 1.

1/2

0107408

0107408

2/2

FIG.2.

FIG.3.

FIG.4.